# EUROPEAN PATENT SPECIFICATION

(11) **EP 1 599 227 B1**
(45) Date of publication and mention of the grant of the patent: **20.01.2010**
(21) Application number: 04714559.4
(22) Date of filing: 25.02.2004
(51) Int. Cl.: A61K 39/395, A61K 39/40, A61K 35/14, C07K 1/00, C07K 14/00, C07K 16/00, C07K 17/00

(54) **METHODS OF IMMUNOMODULATION IN ANIMALS**
VERFAHREN ZUR IMMUNMODULATION BEI TIEREN
PROCEDES D'IMMUNOMODULATION CHEZ DES ANIMAUX

(30) Priority: 25.02.2003 US 375844
(43) Date of publication of application: 30.11.2005
(73) Proprietor: The Lauridsen Group Inc., Ames, Iowa 50010 (US); Campbell, Joy M., Ames, Iowa 50010 (US); Strohbehn, Ronald E., Nevada, Iowa 50201 (US); Weaver, Eric M., Story City, Iowa 50248 (US); Borg, Barton S., Ames, Iowa 50014 (US); Russell, Louis E., Johnston, Iowa 50131 (US); Polo Pozo, Francisco Javier, 08210 Barcelona (ES); Arthington, John D., Punta Gorda, FL 33982 (US); Quigley, III, James D., Ames, IA 50014 (US)
(72) Inventor: CAMPBELL, Joy, M., Ames, Iowa 50010 (US); STROHBEHN, Ronald, E., Nevada, Iowa 50201 (US); WEAVER, Eric, M., Story City, Iowa 50248 (US); BORG, Barton, S., Ames, Iowa 50014 (US); RUSSELL, Louis, E., Johnston, Iowa 50131 (US); POLO POZO, Francisco Javier, Carretera Barcelona, Barbera Del Valles, 08210 Barcelona (ES); ARTHINGTON, John, D., Punta Gorda, Florida 33982 (US); QUIGLEY, James, D., III, Ames, IA 50014 (US)
(74) Representative: Albutt, Jodie
(86) International application number: PCT/US2004/005499
(87) International publication number: WO 2004/075988

(56) References cited:
- WO-A-02/078741
- WO-A-02/078742
- US-A- 4 816 252
- US-A1- 2003 099 633
- US-A1- 2003 103 962
- QUIGLEY J D 3RD ET AL: "Effects of spray-dried animal plasma in milk replacers or additives containing serum and oligosaccharides on growth and health of calves." JOURNAL OF DAIRY SCIENCE FEB 2002, vol. 85, no. 2, February 2002 (2002-02), pages 413-421, XP002450618 ISSN: 0022-0302
- QUIGLEY J D 3RD ET AL: "Effects of spray-dried animal plasma in calf milk replacer on health and growth of dairy calves." JOURNAL OF DAIRY SCIENCE FEB 2003, vol. 86, no. 2, February 2003 (2003-02), pages 586-592, XP002450619 ISSN: 0022-0302
- CAMPBELL J M ET AL: "Effect of spray-dried bovine serum on intake, health, and growth of broilers housed in different environments." JOURNAL OF ANIMAL SCIENCE NOV 2003, vol. 81, no. 11, November 2003 (2003-11), pages 2776-2782, XP002450620 ISSN: 0021-8812
- QUIGLEY J D III ET AL: "Effects of spray-dried animal plasma on apparent digestibility, intake and fecal consistency in adult Beagles." JOURNAL OF DAIRY SCIENCE, vol. 86, no. Supplement 1, 2003, page 259, XP002450621 & JOINT ANNUAL MEETING OF THE AMERICAN DAIRY SCIENCE ASSOCIATION, THE AMERICAN SOCIETY OF ANIMAL SCIEN; PHOENIX, ARIZONA, USA; JUNE 22-26, 2003 ISSN: 0022-0302
- DUST J M ET AL: "Effects of supplemental spray dried plasma on food intake, nutrient digestibility, and gastrointestinal microflora in healthy adult dogs." JOURNAL OF DAIRY SCIENCE, vol. 86, no. Supplement 1, 2003, page 260, XP002450622 & JOINT ANNUAL MEETING OF THE AMERICAN DAIRY SCIENCE ASSOCIATION, THE AMERICAN SOCIETY OF ANIMAL SCIEN; PHOENIX, ARIZONA, USA; JUNE 22-26, 2003 ISSN: 0022-0302
- QUIGLEY J.D. ET AL: 'Effects of Spray-Dried Plasma in the Diets of Companion Animals' PET FOOD FORUM 2002, pages 1 - 22, XP008087150
- 'Endure tm. Product Information and Material Safety Date Sheet' APC, INC. 2002, XP008087149

## Description

### BACKGROUND OF THE INVENTION

The primary source of nutrients for the body is blood, which is composed of highly functional proteins including immunoglobulin, albumin, fibrinogen and hemoglobin. Immunoglobulins are products of mature B cells (plasma cells) and there are five distinct immunoglobulins referred to as classes: M, D, E, A, and G. IgG is the main immunoglobulin class in blood. Intravenous administration of immunoglobulin products has long been used to attempt to regulate or enhance the immune system. Most evidence regarding the effects of intravenous IgG on the immune system suggests the constant faction (Fc) portion of the molecule plays a regulatory function. The specific antigen binding properties of an individual IgG molecule are conferred by a three dimensional steric arrangement inherent in the amino acid sequences of the variable regions of two light and two heavy chains of the molecule. The constant region can be separated from the variable region if the intact molecule is cleaved by a proteolytic enzyme such as papain. Such treatment yields two fractions with antibody specificity (Fab fractions) and one relatively constant fraction (Fc). Numerous cells in the body have distinct membrane receptors for the Fe portion of an IgQ molecule (For). Although some For receptors bind free IgG, most bind it more efficiently if an antigen is bound to the antibody molecule. Binding an antigen results in a configurational change in the Fc region that facilitates binding to the preceptor. A complex interplay of signals provides balance and appropriateness to an immune response generated at any given time in response to an antigen. Antigen specific responses are initiated when specialized antigen presenting cells introduce antigen, forming a complex with the major histocompatibility complex molecules to the receptors of a specific helper inducer T-cells capable of recognizing that complex. IgG appears to be involved in the regulation of both allergic and autoimmune reactions. Intravenous immunoglobulin for immune manipulation has long been proposed but has achieved mixed results in treatment of disease states. A detailed review of the use of intravenous immunoglobulin as drug therapy for manipulating the immune system is described un Vol. 326, No. 2, pages 107-116, New England Journal of Medicine, Dwyer, John M.
QUIGLEY J.D. ET AL, PET FOOD FORUM 2002, pages 1 - 22, discloses effects of spray-dried plasma in the diets of companion animals.

As improvements are made in companion animal diet quality and health care, their life expectancy is Increased. Unfortunately, the geriatric pet population tends to have numerous health concerns, such as a less desirable intestinal microbial balance and diminished immune capacity (Keams et al., 1998; Shultz, 1984). Alleviating these health concerns through dietary modulation is one possible way to improve longevity and quality of life of senior companion animals.

The colonic microflora is proposed to play an important role in the development and maintenance of the host immune system (Gaskins, 2001). In both humans and dogs, an increase in fecal concentrations of potentially harmful bacteria, such as *Clostridium* perfringens, and a reduction in beneficial bacteria, such *as Bifidobacterium,* has been associated with increasing age (Mitsuoka, 1992; Benno et al.,1992). Normal immune responses increase during fetal and early neonatal periods; reach their maximum after puberty, and then decrease markedly with old age (Schultz, 1984). Both the humoral and cellular immune capacities are reduced in older animals compared with adolescent or juvenile animals (Schultz, 1994).

There is a continuing effort and need in the art for improved compositions and methods for immune modulation of animals. Appropriate immunomodulation is essential to improve response to pathogens, vaccinations, for increasing weight gain and improving feed efficiency, improved health and for treatment of immune dysfunction disease states.

The present application describes methods and pharmaceutical compositions for treating animals with immune dysfunction disease states.

The present application describes methods and compositions for immunomodulation of animals including humans for optimizing the response to antigens presented in vaccination protocols.

The present application describes methods to increase weight gain, improve overall health and improve feed efficiency of animals by appropriately modulating the immune system of said animals.

It is an object of the invention to provide a novel pharmaceutical composition comprising purified plasma, components or derivatives thereof, which may be orally administered to create a serum IgG response.

The present application describes methods for modulating the immune system and gut microbial response in animals via a feed supplement.

It is yet another object of the invention to provide methods for dietary modulation to enhance immune function in senior dogs.

Another object of the present invention is to alter colonic microbial populations of senior dogs.

These and other objects of the invention will become apparent from the detailed description of the invention which follows.

### BRIEF SUMMARY OF THE INVENTION

According to an embodiment of the invention, applicants have identified purified and isolated plasma, components, and derivatives thereof, which are useful as a pharmaceutical composition for immune modulation of animals including humans. According to an embodiment, a plasma composition comprising immunoglobulin, when administered orally, induces a lowering of serum IgG levels relative to animals not orally fed immunoglobulin. An orally administered plasma composition comprising immunoglobulin affects the animals overall immune status when exposed to an antigen, vaccination protocols, and for treatment of immune dysfunction disease states.

Applicants have unexpectedly shown that oral administration of plasma protein can induce a change in serum immunoglobulin. This is unexpected as traditionally it was thought that plasma proteins such as immunoglobulins must be introduced intravenously to affect circulating IgG concentration. In contrast, applicants have demonstrated that oral globulin is able to impact circulating serum IgG levels. This greatly simplifies the administration of a dietary modulating compositions, such as immunoglobulin, as these compositions according to the invention, can now be simply added to feedstuff or even water to modulate vaccination, to modulate disease challenge, treat animals with immune dysfunction disease states, enhance an animal's immune function, or alter colonic microbial populations.

Also according to the invention, applicants have demonstrated that modulation of serum IgG impacts the immune system response to stimulation as in vaccination protocols or to immune dysfunction disorders. Modulation of serum IgG, according to the invention allows the animals' immune system to more effectively respond to challenge by allowing a more significant up regulation response in the presence of a disease state or antigen presentation. Further this immune regulation impacts rate and efficiency of gain, as the bio-energetic cost associated with heightened immune function requires significant amounts of energy and nutrients which is diverted from such things as cellular growth and weight gain. Modulation of the immune system allows energy and nutrients to be used for other productive functions such as growth or lactation. See, Buttgerut et al., "Bioenergetics of Immune Functions: Fundamental and Therapeutic Aspects", Immunology Today, April 2000, Vol. 21, No. 4, pp. 192-199.

Applicants have further identified that by oral consumption, the Fc region of the globulin composition is essential for communication and/or subsequent modulation of systemic serum IgG. This is unique, as this is the non-specific immune portion of the molecule which after oral consumption modulates systemic serum IgG without intravenous administration as previously noted (Dwyer, 1992). The antibody specific fractions produced less of a response without the Fc tertiary structure. Additionally, the globulin portion with intact confirmation gave a better reaction than the heavy and light chains when separated therefrom.

The present invention is directed to methods of dietary modulation of animals. More specifically, the present invention is directed to methods to enhance the immune capacity of an animal and alter colonic microbial populations in senior dogs. The method comprises administering to a senior animal an effective amount of a supplement comprising animal plasma from an animal source. As used herein, an effective amount means an amount that will enhance the immune function or alter colonic microbial populations of senior companion animals when administered the supplement in a concentration of 0.5 - 2.0% of the diet.

Also provided is an immune-enhancing animal supplement wherein the supplement comprises an effective amount of an animal protein.

In another embodiment, a plasma composition comprising immunoglobulin, when administered orally, induces a lowering of serum IgG levels relative to animals not orally fed immunoglobulin. An orally administered plasma composition comprising immunoglobulin affects the animal's overall immune status when exposed to an antigen, vaccination protocols, for treatment of immune dysfunction disease states, and altering colonic microbial populations in senior dogs.

### BRIEF DESCRIPTION OF THE DRAWINGS

Figure 1 is a graph depicting the effect of oral administration of plasma protein on antibody responses to a primary and secondary rotavirus vaccination.
Figure 2 is a graph depicting the effect of oral administration of plasma proteins on antibody responses to a primary and secondary PRRS vaccination.
Figures 3A and 3B are graphs depicting the body weight of water treated and plasma treated groups respectively after a respiratory disease challenge.
Figure 4 is a graph depicting the percent of turkeys remaining after the respiratory disease challenge.
Figure 5 is a graph depicting the percent of turkeys remaining before the respiratory disease challenge.
Figure 6 is a graph depicting canine parvovirus hemagluttination titer of senior dogs fed an extended diet supplement with spray dried plasma wherein the values are Ismeans of dogs responding to vaccine challenge, n = 3, 5, 2, and 2 dogs for supplement levels 0, 0.5, 1, and 2%, respectively. NS = not significantly different (P> 0.15).
Figure 7 is a graph depicting total peripheral white blood cell concentration for senior dogs fed an extruded diet supplemented with spray dried plasma wherein the values are Ismeans of dogs responding to vaccine challenge. On days 0, 2,4, 6, 8, and 10, n = 3, 5, 2, and 2 for supplement levels 0, 0.5, 1, and 2%, respectively. On day 20, n = 3, 5, 2, and 1 for supplement levels 0, 0.5,1, and 2%, respectively. NS = not significantly different (P>0.15).

### DETAILED DESCRIPTION OF THE PREFERRED EMBODIMENT

According to the invention, Applicant has provided herein a pharmaceutical composition comprising components purified and concentrated from animal plasma which are useful in practicing the methods of the invention. According to the invention gamma-globulin isolated from animal sources such as serum, plasma, egg, or milk is administered orally in conjunction with vaccination protocols, for treatment of various immune dysfunction disease states to modulate stimulation of the immune system. Quite surprisingly oral administration of this composition has been found to lower serum IgG levels relative to no administration of the pharmaceutical composition. Starting from a less stimulated state, the immune system is able to mount a more aggressive response upon challenge. Furthermore, disease states associated with elevated IgG levels are improved. As used herein with reference to the composition of the invention, the terms "plasma", "globulin", "gamma-globulin", and "immunoglobulin" will all be used. These are all intended to describe a composition purified from animal sources including blood, egg, or milk which retains the Fc region of the immunoglobulin molecule. This also includes transgenic recombinant immunoglobulins purified from transgenic bacteria, plants or animals. This can be administered by spray-dried plasma, or globulin which has been further purified therefrom, or any other source of serum globulin which is available. One such source of purified globulin is NUTRAGAMMAX™ or IMMUNOLIN™ available from Proliant Inc. Globulin may be purified according to any of a number of methods available in the art, including those described in Akita, E.M. and S. Nakai. 1993. Comparison of four purification methods for the production of immunoglobulins from eggs laid by hens immunized with an enterotoxigenic E. coli strain. Journal of Immunological Methods 160:207-214; Steinbuch, M. and R. Audran. 1969. The isolation of IgG from mammalian sera with the aid of caprylic acid. Archives of Biochemistry and Biophysics 134:279-284; Lee, Y., T. Aishima, S. Nakai, and J.S. Sim. 1987. Optimization for selective fractionation of bovine blood plasma proteins using poly(ethylene glycol). Journal of Agricultural and Food Chemistry 35:958-962; Polson, A., G.M. Potgieter, J.F. Langier, G.E.F. Mears, and F.J. Toubert. 1964. Biochem. Biophys. Acta. 82:463-475.

Animal plasma from which gamma globulin may be isolated include pig, bovine, ovine, poultry, equine, or goat plasma. Additionally, applicants have identified that cross species sources of the gamma globulins still provides the effects of the invention.

Concentrates of the product can be obtained by spray drying, lyophylization, or any other drying method, and the concentrates may be used in their liquid or frozen form. The active ingredient may also be microencapsulated, protecting and stabilizing from high temperature, oxidants, pH-like humidity, etc. The pharmaceutical compositions of the invention can be in tablets, capsules, ampoules for oral use, granulate powder, cream, both as a unique ingredient and associated with other excipients or active compounds, or even as a feed additive.

One method of achieving a gamma-globulin composition concentrate of the invention is as follows although the globulin may be delivered as a component of plasma.

The immunoglobulin concentrate is derived from animal blood. The source of the blood can be from any animal that has blood which includes plasma and immunoglobulins. For convenience, blood from beef, pork, and poultry processing plants is preferred. Anticoagulant is added to whole blood and then the blood is centrifuged to separate the plasma. Any anticoagulant may be used for this purpose, including sodium citrate and heparin. Persons skilled in the art can readily appreciate such anticoagulants. Calcium is then added to the plasma to promote clotting, the conversion of fibrinogen to fibrin; however other methods are acceptable. This mixture is then centrifuged to remove the fibrin portion.

Once the fibrin is removed from plasma resulting in serum, the serum can be used as a principal source of Ig. Alternatively, one could also inactivate this portion of the clotting mechanism using various anticoagulants.

The defibrinated plasma is next treated with an amount of salt compound or polymer sufficient to precipitate the albumin or globulin fraction of the plasma. Examples of phosphate compounds which may be used for this purpose include all polyphosphates, including sodium hexametaphosphate and potassium polyphosphate. The globulin may also be isolated through the addition of polyethylene glycol or ammonium sulfate.

Following the addition of the phosphate compound, the pH of the plasma solution is lowered to stabilize the albumin precipitate. The pH should not be lowered below 3.5, as this will cause the proteins in the plasma to become damaged. Any type of acid can be used for this purpose, so long as it is compatible with the plasma solution. Persons skilled in the art can readily ascertain such acids. Examples of suitable acids are HCl, acetic acid, H₂SO₄, citric acid, and H₂PO₄. The acid is added in an amount sufficient to lower the pH of the plasma to the designated range. Generally, this amount will range from a ratio of about 1:4 to 1:2 acid to plasma. The plasma is then centrifuged to separate the globulin fraction from the albumin fraction.

The next step in the process is to raise the pH of the globulin fraction with a base until it is no longer corrosive to separation equipment. Acceptable bases for this purpose include NaOH, KOH, and other alkaline bases. Such bases are readily ascertainable by those skilled in the art. The pH of the globulin fraction is raised until it is within a noncorrosive range which will generally be between 5.0 and 9.0. The immunoglobulin fraction is then preferably microfiltered to remove any bacteria that may be present.

The final immunoglobulin concentrate can optionally be spray-dried into a powder. The powder allows for easier packaging and the product remains stable for a longer period of time than the raw globulin concentrate in liquid or frozen form. The immunoglobulin concentrate powder has been found to contain approximately 35-50% IgG.

In addition to administration with conventional carriers, active ingredients may be administered by a variety of specialized delivery drug techniques which are known to those of skill in the art. The following examples are given for illustrative purposes only and are in no way intended to limit the invention.

Those skilled in the medical arts will readily appreciate that the doses and schedules of the immunoglobulin will vary depending on the age, health, sex, size and weight of the patient rather than administration, etc. These parameters can be determined for each system by well-established procedures and analysis e.g., in phase I, II and III clinical trials.

For such administration the globulin concentrate can be combined with a pharmaceutically acceptable carrier such as a suitable liquid vehicle or excipient and an optional auxiliary additive or additives. The liquid vehicles and excipients are conventional and are commercially available. Illustrative thereof are distilled water, physiological saline, aqueous solutions of dextrose and the like.

In general, in addition to the active compounds, the pharmaceutical compositions of this invention may contain suitable excipients and auxiliaries which facilitate processing of the active compounds into preparations which can be used pharmaceutically. Oral dosage forms encompass tablets, dragees, and capsules.

The pharmaceutical preparations of the present invention are manufactured in a manner which is itself well known in the art. For example the pharmaceutical preparations may be made by means of conventional mixing, granulating, dragee-making, dissolving, lyophilizing processes. The processes to be used will depend ultimately on the physical properties of the active ingredient used.

Suitable excipients are, in particular, fillers such as sugars for example, lactose or sucrose, mannitol or sorbitol, cellulose preparations and/or calcium phosphates, for example, tricalcium phosphate or calcium hydrogen phosphate, as well as binders such as starch, paste, using, for example, maize starch, wheat starch, rice starch, potato starch, gelatin, gum tragacanth, methyl cellulose, hydroxypropylmethylcellulose, sodium carboxymethylcellulose, and/or polyvinyl pyrrolidone. If desired, disintegrating agents may be added, such as the above-mentioned starches as well as carboxymethyl starch, cross-linked polyvinyl pyrrolidone, agar, or alginic acid or a salt thereof, such as sodium alginate. Auxiliaries are flow-regulating agents and lubricants, for example, such as silica, talc, stearic acid or salts thereof, such as magnesium stearate or calcium stearate and/or polyethylene glycol. Dragee cores may be provided with suitable coatings which, if desired, may be resistant to gastric juices.

For this purpose concentrated sugar solutions may be used, which may optionally contain gum arabic, talc, polyvinylpyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropylmethylcellulose phthalate, dyestuffs and pigments may be added to the tablet of dragee coatings, for example, for identification or in order to characterize different combination of compound doses.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, liquid paraffin, or liquid polyethylene glycols. In addition stabilizers may be added.

Oral doses of globulin or plasma protein according to the invention were found to modulate the primary and secondary immune response to rotavirus and PRRS vaccinations by helping to modulate IgG and the immune system.

Methods of the invention also include prevention and treatment of gastrointestinal diseases and infections, malabsorption syndrome, and intestine inflammation, and improving autoimmune states and reduction of systemic inflammatory reactions in humans and animals. The drug compositions, food and dietary preparations would be valid to improve the immune state in humans and animals, for diseases associated with elevated IgG, diseases associated with immune regulatory dysfunction, for the support and treatment of malabsorption processes in humans and animals, and for treatment of clinical situations suffering from malnutritionin humans and animals. Among these malabsorption processes include syndrome of the small intestine, non-treatable diarrhea of autoimmune origin, lymphoma, postgastrectomy, steatorrhea, pancreas carcinoma, wide pancreatic resection, vascular mesentery failure, amyloidosis, scleroderma, eosinophilicenteritis. Clinical situations associated with malnutrition would include ulcerative colitis, Crohn's disease, cancerous cocachexia due to chronic enteritis from chemo or radiotherapy treatment, and medical and infectious pathology comprising severe malabsorption such as AIDS, cystic fibrosis, enterocutaneous fistulae of low debit, and infantile renal failure.

The clinical uses of the composition would typically include disease states associated with immune dysfunction, particularly disease states associated with chronic immune stimulation. Examples of such diseases include but are not limited to myasthenia gravis, multiple sclerosis, lupus, polymyositis, Sjogren's syndrome, rheumatoid arthritis, insulin-dependent diabetes mellitus, bullous pemphigoid, thyroid-related eye disease, ureitis, Kawasaki's syndrome, chronic fatigue syndrome, asthma, Crohn's disease, graft-vs-host disease, human immunodeficiency virus, thrombocytopenia, neutropenia, and hemophilia.

Oral administration of IgG has tremendous advantages over parenteral administration. The most obvious are the risks associated with intravenous administration including: allergic reactions, the increased risk of disease transfer from human blood such as HIV or Hepatitis, the requirement for the same specie source, the cost of administration, and the benefits of oral IgG is greater neutralization of endotoxin and the "basal" stimulation of the immune system; the potential use of xenogeneic IgG. Applicants' invention provides a non-invasive method of modulating the immune response. This can be used to treat autoimmune disorders (e.g., Rhesus reactions, Lupus, rheumatoid arthritis, etc.) and other conditions where immunomodulation, immunosuppression or immunoregulation is the desired outcome (organ transfers, chronic immunostimulatory disorders, etc.).

In another embodiment the invention can be used for oral immunotherapy (using antibodies) as an alternative to IVIG. But, prior to applicants' invention, one could not produce the massive amounts of antibodies required for sustained treatment because IVIG would require human IVIG. With oral administration of antibody, one can use a different specie source, without the threat of allergic reaction. This opens the door to milk, colostrum, serum, plasma, eggs, etc. from pigs, sheep, goats, cattle, etc. as the means of producing the relatively large amounts of immunoglobulin that would be required for sustained treatment.

The oral administration of antibody can:
1) Modulate the immunological response to exposure to a like/similar antigen. The data produced from the immunization of pigs with rotavirus or PRRS show that the oral administration of porcine immunoglobulin modifies the subsequent immune response to antigen administered intramuscularly. Communication occurs via the effects of IgG on the immune cells located in the GI tract (primarily the intestinal epithelium and lymphatic tissue). The plasma administered to the animals traditionally would contain antibody to both PRRS and rotavirus. Previous research has demonstrated that colostrum (maternal antibody) has this same effect when administered prior to gut closure. Applicant has demonstrated that antibody can modulate the immune response in an animal post gut-closure;
2) Serum IgG concentrations are lower with the oral administration of plasma proteins. This effect provides benefits to the prevention or treatment of much different conditions (e.g. Crohn's, IBD, IBS, sepsis, etc.) than the immunosuppressive effects of specific antibodies. This effect is not antibody specific. While not wishing to be bound by any theory it is postulated that plasma proteins can neutralize a significant amount of endotoxin in the lumen of the gut. In the newly weaned pig, that gut barrier function is compromised and will "leak" endotoxin. Endotoxin (LPS) is one of the most potent immunostimulatory compounds known. Thus as a post weaning aid, this invention can improve an animal's response to endotoxin by modulating the immune system preventing overstimulation.

The route of feeding is important to the different effects. Parenteral feeding increases gut permeability and is known to substantially increase the likelihood of sepsis and endotoxemia when compared to parenteral feeding. The oral supply of immunoglobulin improves gut barrier function and reduces the absorption of endotoxin. Diminished absorption of endotoxin would reduce the amount of endotoxin bound in plasma which would increase the plasma neutralizing capacity when compared to control animals.

Applicants' invention discloses immunomodulation, consistent with the observations of the effects of IVIG in the literature. Further, the immunomodulation effect of IgG was observed with different specie sources of IgG administered orally. This is very important to human medicine, particularly for autoimmune conditions (or cases where immunomodulation is desired).

### References:

American Association of Cereal Chemists. (1983) Approved Methods, 8th ed. AACC, St. Paul, MN.
Association of Official Analytical Chemists. (1984) Official Methods of Analysis, 14th ed. AOAC, Washington, DC.
Association of Official Analytical Chemists. (1995) Official Methods of Analysis 15th ed. AOAC, Washington, DC.
Araya-Kojima, T., Yaeshima, T., Ishibashi, N., Shimamura, S. & Hayasawa, H. (1995) Inhibitory effects of Bifidobacterium longum BB536 on harmful intestinal bacteria. Bifidobacteria Microflora 14:59-66.
Bier, M. Aug 1, 2000. Oral immunotherapy of bacterial overgrowth. U.S. Patent No. 6,096,310.
Bridger, J.C. and J.F. Brown. 1981. Development of immunity to porcine rotavirus in piglets protected from disease by bovine colostrum. Infection and Immunity 31:906.
Benno, Y., Nakao, H., Uchida, K. & Mitsuoka, T. (1992) Impact of the advances in age on the gastrointestinal microflora of beagle dogs. J. Vet. Med. Sci. 54:703-706.
Budde, E.F. (1952) The determination of fat in baked biscuit type dog foods. J. Assoc. Off. Agric. Chem., 35, 799-805.
Carmichael, L. E., Joubert, J. C. & Pollock, R. V. H. (1980) Hemagglutination by canine parvovirus: Serologic studies and diagnostic applications. Am. J. Vet. Res. 41:784-791.
Coffey, R.D. & Cromwell, G.L. (1995) The impact of environment and antimicrobial agents on the growth response of early-weaned pigs to spray-dried porcine plasma. J. Anim. Sci. 73:2532-2539.
Cunningham-Rundles, S. 1994. Malnutrition and gut immune function. Current Opinion in gastroenterology. 10:644-670.
Dwyer, J.M. 1992. Drug Therapy. Manipulating the Immune system with Immune Globulin. N.E.J.M. 326:107-116.
Eibl, M.M., H.M. Wolf, H. Furnkranz, and A Rosenkranz. 1988. Prevention of necrotizing enterocolitis in low-birth-weight infants by IgA-IgG feeding. N.E.J.M. 319:1-7.
Food and Drug Administration (1992) Method #196. In: Bacteriological Analytical Manual, 7th ed., p. 506, Arlington, VA.
Gaskins, H. R. (2001) Intestinal bacteria and their influence on swine growth. In: Swine Nutrition. (Lewis, A. J. and Southern, L. L., eds.), pp. 585-608. CRC Press, Boca Raton, FL.
Gibson, G. R. & Wang, X. (1994) Regulatory effects of bifidobacteria on the growth of other colonic bacteria. J. Appl. Bacteriol. 77:412-420.
Gibson, G. R. & Roberfroid, M. B. (1995) Dietary modulation of the human colonic microbiota: Introducing the concept of prebiotics. J. Nutr. 125:1401-1412.
Hara, J., Li, S. T., Sasaki, M., Maruyama, T., Terada, A., Ogata, Y., Fujita, K., Ishigami, H., Hara, K., Fujimori, I. & Mitsuoka, T. (1994) Effective dose of lactosucrose on fecal flora and fecal metabolites of humans. Bifidobacteria Microflora 13 :51-63.
Hardic, W.R. 1984. Oral immune globulin. U.S. Patent No. 4,477,432. Filed April 5, 1982.
Hammarstrom, L., A. Gardulf, V. Hammarstrom, A. Janson, K. Lindberg, and C.I. Edvard Smith. 1994. Systemic and topical immunoglobulin treatment in immunocompromised patients. Immunological Reviews 139:43-70.
Heneghan, J.B. 1984. Physiology of the alimentary tract. In: Coats, M.E., B.E. Gustafsson eds. The germ-free animal in biomedical research. London: Laboratory Animals Ltd. Pp. 169-191.
Henry, C. and N. Herne. 1968. J. Exp. Med. 128:133-152.
Karlsson, M.C.I., S. Wemersson, T. Diaz de stahl, S. Gustavsson, and B. Heyman. 1999. Efficient IgG-mediated suppression of primary antibody responses in Fcy receptor-deficient mice. Proc. Natl. Acad. Sci. 96:2244-2249.
Kearns, R. J., Hayek, M. G. & Sunvold, G. A. (1998) Microbial changes in aged dogs. In: Recent Advances in Canine and Feline Nutrition: 1998 lams Nutr. Symp. Proc. pp. 337-351. (Reinhart, G. A. and Carey, D. P., Eds.), Orange Frazer Press, Wilmington, OH.
Klobasa, F., J.E. Butler, and F. Habe, 1990. Maternal-neonatal immunoregulation: suppression of de novo synthesis of IgG and IgA, but not IgM, in neonatal pigs by bovine colostrum, is lost upon storage. Am. J. Vet. Res. 51:14407-1412.
McCracken, B.A., M.E. Spurlock, M.A. Roos, F.A. Zuckermann, and H. Rex Gaskins. Weaning anorexia may contribute to local inflammation in the piglet small intestine. J. Nutr. 129:613.
Mitsuoka, T. (1982) Recent trends in research on intestinal flora. Bifidobacteria Microflora 1:3-24.
Mitsuoka, T. (1992) Intestinal flora and aging. Nutr. Rev. 40:438-446.
Muñoa, F. J. & Pares, R. (1988) Selective medium for isolation and enumeration of Bifidobacterium spp. Appl. Environ. Microbiol. 54:1715-1718.
Mietens, C. and H. Keinhorst. 1979. Treatment of infantile E. coli gastroenteritis with specific bovine anti-E. coli milk immunoglobulins. Eur. J. Pediatr. 132:239-252.
Murphy, F. A., Gibbs, E. P. J., Horzinek, M. C. & Studdert, M. J. (editors). 1999. Chapter 21: Parvoviridae. In: Veterinary Virology, 3rd Ed., pp 352-353. Academic Press, San Diego, CA
O'Gormon, P., D.C. McMillan, and C.S. McArdle. 1998. Impact of weight loss, appetite, and the inflammatory response on quality of life in gastrointestinal cancer patients. Nutrition and Cancer 32(2):76-80.
Rowlands, B.J. and K.R. Gardiner. 1998. Nutritional modulation of gut inflammation. Proceedings of the Nutrition Society 57:395-401.
Schultz, R. D. (1984) The effects of aging on the immune system. In: 33rd Annu. Gaines Symp. Canine Geriatrics. Comp. Cont. Edu. Pract. Vet. 6:1096-1105.
Sharma, R., U. Schumacher, V. Ronaasen, and M. Coates. 1995. Rat intestinal mucosal responses to a microbial flora and different diets. Gut 36:209-214.
Strasser, A., Niedermuller, H., Hofecker, G. & Laber, G. (1993) The effect of aging on laboratory values in dogs. J. Vet. Med. A. 40:720-730.
Van der Poll, T., M. Levi, C.C. Braxton, S.M. Coyle, M. Roth, J.W. Ten Cate, and S.F. Lowry. 1998. Parenteral nutrition facilitates activation of coagulation but not fibrinolysis during human endotoxemia. J. Infect. Dis. 177:793-795.
Wolf, H.M. and M.M. Eibl. 1994. The anti-inflammatory effect of an oral immunoglobulin (IgA-IgG) preparation and its possible relevance for the prevention of necrotizing enterocolitis. Acta Pediatr. Suppl. 396:37-40.
Skarnes, R.C. 1985, In vivo distribution and detoxification of endotoxins. In: Proctor, R.A. (ed): Handbook of Endotoxin, Vol. 3, Pp. 56-81.
Zhang, G.H., L. Baek, T. Bertelsen and C. Kock. 1995. Quantification of the endotoxin-neutralizing capacity of serum and plasma. APMIS 103:721-730.

### EXAMPLE 1

### Preferred Manufacturing Method For Globulin Concentrate

The following illustrates a preferred method of manufacturing the globulin concentrate of the present invention:

| |
|---|
| Plasma |
| ↓ |
| Recalcification of plasma |
| ↓ |
| Centrifuge to remove fibrin |
| ↓ |
| Filter sock |
| ↓ |
| salt precipitation |
| centrifuge |
| ↙↘ |
| **Globulin Rich Fraction Discard** |

### EXAMPLE 2

### Necessity of Intact Globulin

Previous research demonstrates that oral plasma consumption improves weanling pig performance (Coffey and Cromwell, 1995). Data indicates that the high molecular weight fraction present in plasma influences the performance of the pig (Cain, 1995; Owen et al, 1995; Pierce et al., 1995, 1996; Weaver et al., 1995). The high molecular weight fraction is composed primarily of IgG protein. Immunoglobulin G protein is approximately 150,000 MW compound consisting of two 50,000 MW polypeptide chains designated as heavy chains and two 25,000 MW chains, designated as light chains (Kuby, 1997). An approach to hydrolysis of intact IgG has been demonstrated in the lab with the enzyme pepsin. A brief digestion with pepsin enzyme will produce a 100,000 MW fragment composed of two Fab-like fragments (Fab = antigen-binding). The Fc fragment of the intact molecule is not recovered as it is digested into multiple fragments (Kuby, 1997). A second type of processing of the globulin-rich concentrate is by disulfide bond reduction with subsequent blocking to prevent reformation of disulfide bonds. The resulting reduced sections from the globulin molecule are free intact heavy and light chains.

In the first example the objective was to quantify the impact by oral consumption of different plasma fractions and pepsin hydrolyzed plasma globulin on average daily gain, average daily feed intake, intestinal morphology, blood parameters, and intestinal enzyme activity in weanling pigs.

### Materials and Methods

*Animals and Diets.* Sixty-four individually penned pigs averaging 6.85 kg body weight and 21 d of age were allotted to four dietary treatments in a randomized complete block design. Two rooms of 32 pens each were used. The nursery rooms previously contained animals from the same herd of origin and were not cleaned prior to placement of the test animals to stimulate a challenging environment. Pigs were given ad libitum access to water and feed.

Dietary treatments are represented in Table 1 consisting of: 1) control; 2) 6% spray-dried plasma; 3) 3.6% spray-dried globulin; and 4) 3.6% spray-dried pepsin digested globulin. Diets are corn-soybean meal-dried whey based replacing menhaden fishmeal with plasma on an equal protein basis. Plasma fractions were included, relative to plasma, on an equal plasma fraction basis. Diets contained 1.60% lysine were formulated to an ideal amino acid profile (Chung and Baker, 1992). Diets were pelleted at 130°F or less and were fed from d 0-14 post-weaning.

*Collection of Data.* Individual pig weights were collected on d 0, 2, 4, 6, 8, 10, 12, and 14 post-weaning. Feed intake and diarrhea score were collected daily from d 0 to 14 post-weaning. Blood was collected d 0,7, and 14 post-weaning. The blood was centrifuged and serum was frozen for subsequent analysis. Upon completion of the study (d 14), six randomly selected pigs/treatment were sacrificed to obtain samples for measurement of villous height, crypt depth, intestinal enzyme activity, and organ weights (intestine, liver, lung, heart, spleen, thymus, kidney, stomach, and pancreas). Immediately after euthanasia, the body cavity was opened and the ileal-cecal juncture was located. The small intestine was removed and dissected free of mesenteric attachment. One meter cranial to the ileal-cecal juncture, 10 cm of intestine (ileum) was removed and fixed in phosphate-buffered formalin for subsequent histology measurements. From the midsection of the duodenum, the mucosa was scraped, weighed, and frozen for subsequent enzymatic analysis.

*Histology.* The jejunal samples were paraffin embedded and stained with hematoxylin and eosin (H&E) and were analyzed using light microscopy to measure crypt depth and villous height. Five sites were measured for crypt depth and villous height on each pig.

*Enzyme analysis.* Lactase and maltase activity were measured on the mucosal scrapings according to Dahlqvist, 1964.

*Serum analysis.* Total protein and albumin were analyzed according to ROCHE Diagnostic kits for a COBAS MIRA system. Serum IgG was analyzed according to Etzel et al. (1997).

*Statistical Analysis.* Data were analyzed as a randomized complete block design. Pigs were individually housed and the pen was the experimental unit. Analysis of variance was performed using the GLM procedures of SAS (SAS/STAT Version 6.11 SAS Institute, Cary, NC). Model sum of squares consisted of block and treatment, using initial weight as a covariate. Least squares means for treatments are reported.

### Results

Average daily gain (ADG) and average daily feed intake (ADFI) are presented in Table 2. No differences were noted for ADG or ADFI from d 0-6. From d 0-14, plasma and globulin improved (P <0.05) ADG and ADFI compared to the control, while the pepsin digested globulin treatment was intermediate. Organ weights were recorded and expressed as g/kg of body weight (Table 3). No differences were noted in heart, kidney, liver, lung, small intestine, stomach, thymus, or spleen; however, pancreas weight was increased (P <0.05) due to inclusion of globulin and pepsin digested globulin compared to the control. The plasma treatment was intermediate. Blood parameters are presented in Table 4. Compared to the control, serum IgG of globulin fed pigs (d 14) was lower (P <0.08), while that of the plasma and pepsin digested globulin treatments were intermediate. No differences (P >0.10) were noted in total protein. Serum albumin was increased (P <.08) on d 14 with the globulin and plasma treatment compared to the control, while that of the pepsin digested globulin group was intermediate. Enzyme activity, intestinal morphology, and fecal score are presented in Table 5. No differences (P >0.10) were noted in villous height and crypt depth. Duodenal lactase and maltase activity was increased (P <0.07) due to consumption of pepsin digested globulin compared to the control diet, while the other dietary treatments were intermediate. The fecal score was reduced (P <0.07; respresenting a firmer stool) due to the addition of pepsin digested globulin compared to the control while the fecal score of and plasma while globulin was intermediate.

### Tables

**Table 1. Composition of experimental diets (as fed, %).^{a}**

| Ingredients | Control | Plasma | Globulin | Pepsin Digested Globulin |
|---|---|---|---|---|
| Corn | 42.932 | 43.012 | 42.962 | 42.957 |
| 47% SBM | 23.000 | 23.000 | 23.000 | 23.000 |
| Dried Whey | 17.000 | 17.000 | 17.000 | 17.000 |
| Menhaden Fishmeal | 8.500 | | 3.400 | 3.400 |
| Plasma | | 6.000 | | |
| Globulin | | | 3.600 | |
| Pepsin Digested | | | | 3.600 |
| Globulin | | | | |
| Soy Oil | 4.300 | 5.100 | 4.800 | 4.800 |
| Lactose | 2.118 | 2.118 | 2.118 | 2.118 |
| 18.5% Dical | 0.400 | 1.700 | 1.150 | 1.150 |
| Limestone | 0.070 | 0.435 | 0.290 | 0.290 |
| Zinc Oxide | 0.400 | 0.400 | 0.400 | 0.400 |
| Mecadox | 0.250 | 0.250 | 0.250 | 0.250 |
| Salt | 0.250 | 0.250 | 0.250 | 0.250 |
| Premix | 0.400 | 0.400 | 0.400 | 0.400 |
| L-Lysine HCL | 0.250 | 0.195 | 0.290 | 0.290 |
| L-Threonine | 0.090 | | | |
| DL-Methionine | 0.040 | 0.140 | 0.090 | 0.095 |

| | | | | |
|---|---|---|---|---|
| ^{a} Diets were formulated to contain 1.60% lysine, 0.48% methionine, 14% lactose, 0.8% calcium, and 0.7% phosphorus and fed from d 0 to 14 post-weaning. | | | | |

**Table 2. Effect of spray-dried plasma and plasma fractions on average daily gain and feed intake (kg/d).¹**

| Treatment | Control | Plasma | Globulin | Pepsin Digested Globulin | SEM |
|---|---|---|---|---|---|
| ADG, kg/d | | | | | |
| D 0-6 | 0.037 | 0.094 | 0.080 | 0.073 | 0.029 |
| D 0-14 | 0.169^{a} | 0.242^{b} | 0.234^{b} | 0.222^{ab} | 0.025 |
| | | | | | |
| ADFI, kg/d | | | | | |
| D 0-6 | 0.104 | 0.134 | 0.132 | 0.128 | 0.018 |
| D 0-14 | 0.213^{a} | 0.276^{b} | 0.278^{b} | 0.254^{ab} | 0.021 |
| | | | | | |

| | | | | | |
|---|---|---|---|---|---|
| ¹Values are least squares means with 16 pigs/treatment. ^{ab}Means within a row without common superscript letters are different (P<0.10). | | | | | |

**Table 3. Effect of spray-dried plasma and plasma fractions on organ weights (g/kg body weight)¹**

| Organ Weights, g/kg BW | Control | Plasma | Globulin | Pepsin Digested Globulin | SEM |
|---|---|---|---|---|---|
| Intestine | 44.21 | 50.65 | 50.34 | 44.71 | 3.43 |
| Liver | 32.34 | 31.20 | 30.23 | 32.27 | 1.42 |
| Spleen | 1.74 | 1.83 | 1.81 | 2.06 | 0.16 |
| Thymus | 1.45 | 1.39 | 1.32 | 1.36 | 0.20 |
| Heart | 4.93 | 4.89 | 4.94 | 4.73 | 0.22 |
| Lung | 11.26 | 11.28 | 12.14 | 11.95 | 1.03 |
| Stomach | 6.96 | 7.06 | 6.61 | 6.84 | 0.32 |
| Kidney | 4.76 | 5.75 | 5.66 | 5.45 | 0.47 |
| Pancreas | 1.93^{a} | 2.20^{ab} | 2.42^{b} | 2.34^{b} | 0.11 |

| | | | | | |
|---|---|---|---|---|---|
| ¹Values are least squares means of 6 pigs/treatment. ^{ab}Means within a row without common superscript letters are different (P<0.05). | | | | | |

**Table 4. Effect of spray-dried plasma and plasma fractions on blood parameters.^{1,2}**

| | Control | Plasma | Globulin | Pepsin Digested Globulin | SEM |
|---|---|---|---|---|---|
| IgG, mg/mL | | | | | |
| D0 | 4.84^{a} | 5.70^{b} | 4.83^{a} | 5.05^{ab} | 0.34 |
| D7 | 4.98 | 4.71 | 4.66 | 4.96 | 0.17 |
| D14 | 4.88^{b} | 4.43^{ab} | 4.30^{a} | 4.54^{ab} | 0_{.}24 |

| Total Protein, g/dL | | | | | |
|---|---|---|---|---|---|
| D0 | 4.55 | 4.59 | 4.54 | 4.65 | 0.07 |
| D7 | 4.39 | 4.37 | 4.35 | 4.47 | 0.08 |
| D14 | 4.22 | 4.30 | 4.29 | 4.20 | 0.07 |

| Albumin, g/dL | | | | | |
|---|---|---|---|---|---|
| D0 | 3.03 | 3.02 | 3.11 | 3.09 | 0.06 |
| D7 | 2.98 | 3.03 | 3.02 | 3.01 | 0.06 |
| D14 | 2.61^{a} | 2.78^{b} | 2.80^{b} | 2.71^{ab} | 0.07 |

| | | | | | |
|---|---|---|---|---|---|
| ¹Values are least squares means of 16 pigs/treatment. ²Day 0 used as a covariate for analysis on D7 and D14. ^{ab}Means within a row without common superscript letters are different (P<0.08). | | | | | |

**Table 5. Effect of spray-dried plasma and plasma fractions on enzyme activities, intestinal morphology, and fecal score.¹**

| | Control | Plasma | Globulin | Pepsin Digested Globulin | SEM |
|---|---|---|---|---|---|
| Maltase, umol/mg prot/hr | 7.97^{a} | 11.08^{ab} | 10.93^{ab} | 13.30^{b} | 1.93 |
| Lactase, umol/mg prot/hr | 1.14^{a} | 1.57^{ab} | 1.55^{ab} | 2.15^{b} | 0.31 |
| Villous Height, micron | 378.7 | 370.7 | 374.0 | 387.7 | 34.4 |
| Crypt Depth, micron | 206.3 | 191.0 | 195.0 | 192.7 | 9.3 |
| Fecal Score | 5.12^{b} | 5.06^{b} | 4.19^{ab} | 2.88^{a} | 0.65 |

| | | | | | |
|---|---|---|---|---|---|
| ¹Values are least squares means of 6 pigs/treatment ^{ab}Means within a row without common superscript letters are different (P<0.07) | | | | | |

### EXAMPLE 3

### Quantity and Impact of Dietary Inclusion of Variable Plasma Fractions

In the second experiment the objective was to quantify the impact of dietary inclusion of different plasma fractions and the effect of separating the heavy and light chains of the IgG on average daily gain, average daily feed intake, organ weights, and blood parameters of weanling pigs.

### Materials and Methods

*Animals and Diets.* Ninety-six individually penned pigs averaging 5.89 kg body weight and 21 d of age were allotted to four dietary treatments in a randomized complete block design. The animals were blocked by time between 3 unsanitized nursery rooms. Pigs were given ad libitum access to water and feed.

Dietary treatments (Table 6) consisted of: 1) control; 2) 10% spray-dried plasma; 3) 6% spray-dried globulin; and 4) 6% globulin-rich material treated to reduce the disulfide bonds of the IgG molecule (H + L). Diets were corn-soybean meal-dried whey based replacing soybean meal with plasma on an equal lysine basis. The plasma fractions were added relative to plasma on an equal plasma fraction basis. Diets contained 1.60% lysine and were formulated to an ideal amino acid profile (Chung and Baker, 1992). Diets were meal form and fed from d 0-14 post-weaning.

*Collection of Data.* Individual pig weights were collected on d 0, 2, 4, 6, 8, 10, 12, and 14 post-weaning. Feed intake and diarrhea score were collected daily from d 0 to 14 post-weaning. Blood was collected on d 0, 7, and 14 post-weaning. The blood was centrifuged and serum samples were frozen for subsequent analysis. Upon completion of the study (d 14), nine pigs/treatment were sacrificed to obtain organ weights (intestine, heart, liver, spleen, thymus, lung, kidney, stomach, and pancreas).

*Serum Analysis.* Total protein, albumin, and urea nitrogen were analyzed according to ROCHE Diagnostic kits for a COBAS MIRA system. Serum IgG was analyzed according to Etzel et al. (1997).

*Statistical Analysis.* Data were analyzed as a randomized complete block design using the GLM procedures of SAS (SAS/STAT Version 6.11 SAS Institute, Cary NC). Pigs were individually housed and the pen was the experimental unit. Model sum of squares consisted of block and treatment, using initial weight as a covariate. Least squares means for treatments are reported.

### Results

From d 0-6 (Table 7), plasma increased (P <0.10) ADFI compared to control and H+L, while the globulin was intermediate. From d 7-14, plasma increased (P <0.10) ADFI compared to control and H+L treatments. Average daily feed intake of globulin fed pigs was increased compared to the control. From d 0-14, plasma and globulin increased (P <0.10) ADFI compared to the control and H+L dietary treatments. Average daily gain is presented in Table 8. Average daily gain was similar to ADFI for d 0-6. From d 7-14 and 0-14, plasma and globulin increased (P <0.10) ADG compared to the control, while H+L was intermediate. Blood parameters are presented in Table 9. Serum IgG and urea nitrogen (d 14) were lower (P<0.05) by the dietary inclusion of plasma and globulin compared to the control. The effect of H+L was intermediate. Dietary treatment had no effect on serum protein. Serum albumin (d 7) was decreased (P <0.05) due to inclusion of plasma compared to the other dietary treatments. No differences were noted in fecal score. Intestinal length and organ weights are presented in Table 10. No differences were noted in organ weights or intestinal length due to dietary treatment.

### Tables

**Table 6. Composition of experimental diets (as fed. %)¹**

| Ingredients | Control | Plasma | Globulin | H + L |
|---|---|---|---|---|
| Corn | 37.937 | 44.96 | 40.006 | 40.034 |
| 47% Soybean Meal | 18 | 18 | 18 | 18 |
| Dried Whey | 14 | 14 | 14 | 14 |
| Lactose | 6.253 | 6.253 | 6.253 | 6.253 |
| Plasma | | 10 | | |
| Globulin | | | 6 | |
| H+L | | | | 6 |
| Soy Protein | 17.31 | | 9.07 | 9.07 |

| Concentrate | | | | |
|---|---|---|---|---|
| Soy Oil | 3.219 | 3.047 | 3.187 | 3.186 |
| 18.5% Dical | 1.79 | 1.493 | 2.133 | 2.146 |
| Limestone | 0.562 | 0.354 | 0.46 | 0.42 |
| Premix | 0.55 | 0.55 | 0.55 | 0.55 |
| Salt | 0.15 | 0.15 | 0.15 | 0.15 |
| DL-Methionine | 0.083 | 0.152 | 0.092 | 0.096 |
| L-Lysine HCL | 0.146 | 0.041 | 0.099 | 0.095 |

| | | | | |
|---|---|---|---|---|
| ¹Diets were formulated to contain 1.60% lysine, 0.48% methionine, 16% lactose, 0.9% calcium, and 0.8% phosphorus and fed from d 0 to 14 post-weaning. | | | | |

**Table 7. Effect of spray-dried plasma and plasma fractions on average daily feed intake (g/d)_{.}¹**

| | Control | Plasma | Globulin | H + L | SEM |
|---|---|---|---|---|---|
| ADFI, g/d | | | | | |
| D 0-6 | 102.82^{a} | 152.43⁶ | 128.53^{ab} | 114.50^{a} | 13.44 |
| D7-14 | 280.74^{a} | 413.57^{c} | 379.21^{bc} | 319.06^{ab} | 29.07 |
| D 0-14 | 193.94^{a} | 284.83^{b} | 258.55^{b} | 216.83^{ab} | 16.69 |

| | | | | | |
|---|---|---|---|---|---|
| ¹ Values are least squares means of 24 pigs/treatment. ^{abc}Means within a row without common superscript letters are different (P <0.10). | | | | | |

**Table 3. Effect of spray-dried plasma and plasma fractions on average daily gain (g/d).¹**

| | Control | Plasma | Globulin | H + L | SEM |
|---|---|---|---|---|---|
| ADG, g/d | | | | | |
| D 0-6 | -41.05^{a} | 27.23^{b} | -1.23^{ab} | -21.86^{a} | 20.26 |
| D 7-14 | 199.38^{a} | 282.46^{b} | 302.22^{b} | 255.12^{ab} | 26.40 |
| D 0-14 | 96.34^{a} | 173.07^{b} | 172.17^{b} | 136.42^{ab} | 20.56 |

| | | | | | |
|---|---|---|---|---|---|
| ¹ Values are least squares means of 24 pigs/treatment. ^{abc}Means within a row without common superscript letters are different (P <0.10). | | | | | |

**Table 9. Effects of spray-dried plasma fractions on blood parameters,^{1,2}**

| | Control | Plasma | Globulin | H + L | SEM |
|---|---|---|---|---|---|
| IgG, g/dL | | | | | |
| D 0 | 0.674 | 0.664 | 0.584 | 0.661 | 0.037 |
| D 7 | 0.668 | 0.643 | 0.624 | 0.673 | 0.021 |
| D 14 | 0.631^{b} | 0.555^{a} | 0.545^{a} | 0.596^{ab} | 0.022 |
| Urea N. mg/dL | | | | | |
| D 0 | 8.53 | 9.78 | 9.94 | 9.87 | 0.68 |
| D 7 | 17.55^{b} | 14.65^{a} | 16.48^{ab} | 17.56^{b} | 1.01 |
| D 14 | 17.57^{c} | 10:48^{a} | 14.73^{b} | 15.56^{bc} | 0.87 |
| Total Protein, g/dL | | | | | |
| D 0 | 4.58 | 4.46 | 4.56 | 4.56 | 0.076 |
| D 7 | 4.69 | 4.60 | 4.53 | 4.74 | 0.106 |
| D 14 | 4.55 | 4.49 | 4.59 | 4.49 | 0.080 |
| Albumin, g/dL | | | | | |
| D 0 | 2.69 | 2.64 | 2.75 | 2.69 | 0.069 |
| D 7 | 2.92^{b} | 2.79^{a} | 2.92^{b} | 2.94^{b} | 0.045 |
| D 14 | 2.83 | 2.76 | 2.86 | 2.80 | 0.060 |

| | | | | | |
|---|---|---|---|---|---|
| ¹Values are least squares means of 24 pigs/treatment. ²Day 0 used as a covariate for analysis on D7 and D14. ^{abc}Means within a row without common superscript letters are different (P <0.05). | | | | | |

**Table 10. Effect of spray-dried plasma and plasma fractions on intestinal length (inches) and organ weights (g/kg body weight)¹**

| | Control | Plasma | Globulin | H + L | SEM |
|---|---|---|---|---|---|
| Int. length, inch | 358.67 | 368.33 | 359.33 | 358.56 | 13.05 |
| Organ weight, g/kg BW | | | | | |
| Intestine | 41.48 | 41.79 | 42.82 | 41.04 | 2.16 |
| Liver | 29.61 | 32.61 | 32.29 | 31.09 | 1.10 |
| Spleen | 2.05 | 2.32 | 2.44 | 2.17 | 0.22 |
| Thymus | 1.15 | 1.45 | 1.15 | 1.15 | 0.14 |
| Heart | 6.12 | 6.14 | 5.77 | 5.80 | 0.22 |
| Lung | 12.24 | 12.33 | 13.65 | 11.63 | 0.74 |
| Stomach | 9.26 | 9.14 | 10.08 | 10.08 | 0.58 |
| Kidney | 6.18 | 6.57 | 6.10 | 6.30 | 0.21 |
| Pancreas | 2.70 | 2.61 | 2.54 | 2.70 | 0.11 |

| | | | | | |
|---|---|---|---|---|---|
| ¹Values are least squares means of 9 pigs/treatment. | | | | | |

### Discussion

Consistent with published research (Coffey and Cromwell, 1995) these data indicate that when included in the diet plasma and globulin increase performance (ADG, ADFI) compared to the control. The pepsin digested globulin and H+L fraction resulted in an intermediate improvement in performance. Enzyme activity (lactase and maltase) were increased and fecal score was improved with the addition of all plasma fractions (plasma, globulin, pepsin digested globulin, H&L) compared to the control.

Serum IgG concentration and BUN were lower after consumption of plasma or globulin treatments compared to the control, pepsin digested globulin or H&L. The ability of oral plasma or globulin administration to elicit a systemic response as demonstrated by lower serum IgG compared to the control was unexpected.

The noted differences between plasma and globulin fractions compared to the pepsin digested globulin or H+L is that the tertiary structure of the Fc region is intact in the plasma and globulin fractions only. The pepsin digested globulin has the Fc region digested, while in the H+L fraction, the Fc region remains intact but without tertiary confirmation. The Fab region is still intact in the pepsin digested globulin. The variable region is still able to bind antigen in the H+L preparation (APC, unpublished Data). Thus, the results indicate the antibody-antigen interaction (Fab region) is important for local effects (reduced fecal score, increased lactase and maltase activity), while the intact Fab and Fc region of plasma and globulin fractions is important to modulate the systemic serum IgG response.

### EXAMPLE 4

### Effect of Oral Doses of Plasma Protein on Active Immune Responses to Primary and Secondary Rotavirus and PRRS Vaccinations in Baby Pigs

### Overview

To examine the influence of supplemental plasma protein on active immune responses following primary and secondary rotavirus and PRRS vaccinations.

### Methods

Ten sows induced to farrow at a common time were utilized. Treatments were assigned randomly within each litter. Treatment delivery occurred twice weekly (3 or 4 day intervals) via a stomach tube applicator. A series of 7 applications occurred prior to the final vaccination and weaning. Treatments consisted of: control (10 mL saline) and plasma IgG (0.5 g delivered in a final volume of 8 mL). All pigs received a primary vaccination (orally = rotavirus; injection = PRRS) 10 days prior to weaning. A secondary vaccination was given at the time of weaning via intramuscular injection. Blood samples were collected prior to the primary vaccination (10 d prior to weaning), prior to the secondary vaccination (at weaning), and on 3 day intervals until 12 days post-weaning.

### Results

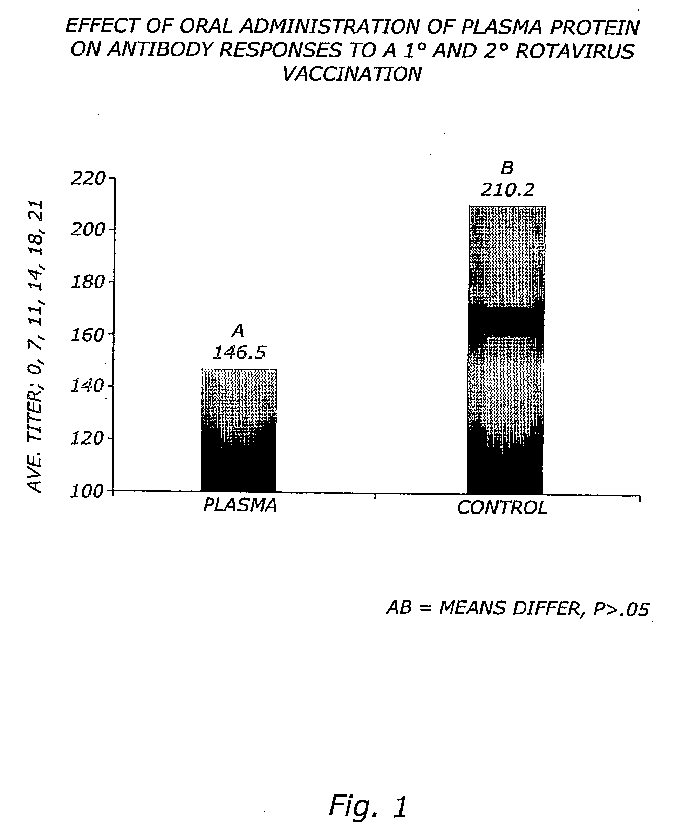

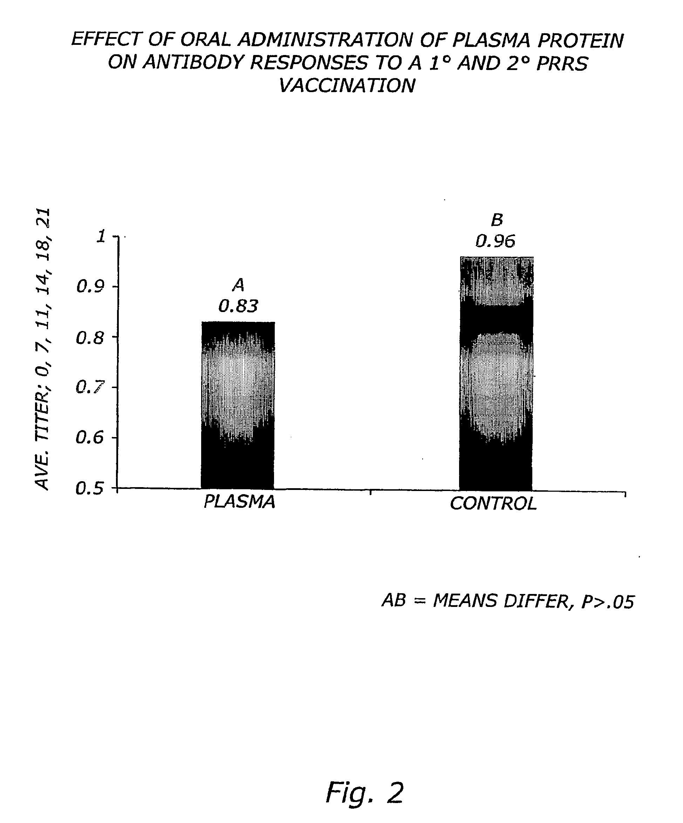

Pigs dosed with plasma protein experienced significant (P<.05) decreases in specific antibody titers following booster vaccination. This response was seen for both rotavirus (Figure 1) and PRRS (Figure 2) antibody titers.

### Discussion

These data provide an excellent indication of the effect of oral plasma protein in the young pig. Immune activation acts as a large energy and nutrient sink. When the immune system is activated energy and nutrients are funneled into the production of immune products (immunoglobulin, cytokines, acute phase proteins, etc.) and away from growth. Oral plasma may modulate the immune system, thereby allowing energy and nutrients to be redirected to other productive functions such as growth.

### EXAMPLE 5

### Evaluation of Plasma Delivered Via Water in Turkeys Under Disease Challenge

### Overview

To evaluate blood or fractions thereof such as serum, plasma or portions purified therefrom preferably containing immunoglobulin, when administered to animals, in particular poultry, and specifically to turkeys via their water, effects death loss in a positive manner when the turkeys are disease challenged. The invention demonstrates improvement in performance of turkeys specifically during the starting period if they have consumed plasma proteins in the water. Overall, delivery of plasma proteins via the water increases feed efficiency and percent remaining (survival) after respiratory challenge and aids in starting turkeys.

### Materials and Methods

Eighty male one day old Nicholas turkey poults were randomly assigned to water treatments. Initial body weight was 59 g. Treatments were applied in a factorial design consisting of 1) disease challenge or no disease challenge and 2) plasma treated water or regular water. The turkey poults were housed as 6 or 7 turkeys per pen utilizing a total of 12 floor pens. The challenge turkeys were separated from the non-challenge turkeys to alleviate cross contamination. Body weight, feed intake and water intakes were measured daily. Turkeys were offered commercially available diets. Fresh water treatments were offered daily. The plasma concentrations in the treated water was altered regularly consisting of 1.3%, 0.65%, 0.325%, and 1.3% for d 0-7, 7-14, 14-21, and 21-49, respectively. The turkeys were challenged on d 35 with pastuerella to induce a respiratory challenge. Clinical signs and death loss were recorded daily from d 0-49. On d 49, the study was terminated and all turkeys were necropcied.

Data were analyzed as a factorial design using the GLM procedures of SAS (SAS/STAT Version 8, SAS Institute, Cary, NC). Model sum of squares consisted of challenge and water treatment. Least squares means are reported. Death loss after challenge was analyzed using survival analysis of SAS.

### Results

Performance data before challenge is presented in Table 11. Since the turkeys were not challenged prior to d 35, only main effects are reported. Inclusion of plasma via the water increased (P < 0.001) average daily gain (ADG) from d 0-7, while no further improvements were noted in gain to d 35. No differences (P > 0.05) were noted in average daily feed intake (ADFI) from d 0-35. Water disappearance was increased (P < 0.05) from d 0-7, 0-14, and 0-21 from consumption of plasma via the water compared to the controls fed untreated water. Feed efficiency (G/F) was increased (P < 0.05) from d 0-7, 7-14, 0-14, and 0-28 from due to consumption of plasma treated water compared to untreated water differences (P > 0.05) were noted in G/F and water disappearance during the remainder of the study till d 35. Performance data after challenge is presented in Table 12. No differences (P > 0.05) were noted in ADG, ADFI, and water disappearance from consumption of plasma treated water compared to treated water for challenge or unchallenged groups. Feed efficiency was improved (P < 0.05) in challenge turkeys from d 35-42 and d 35-49 due to consumption of plasma treated water compared to untreated water; while, the no differences (P > 0.05) were noted in unchallenged turkeys due to consumption of plasma treated water.

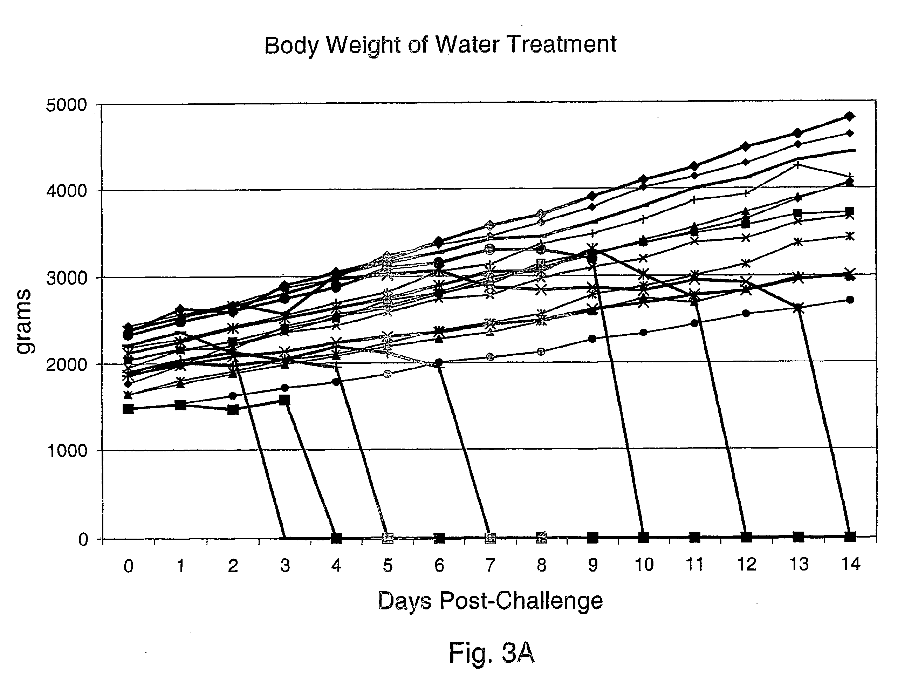

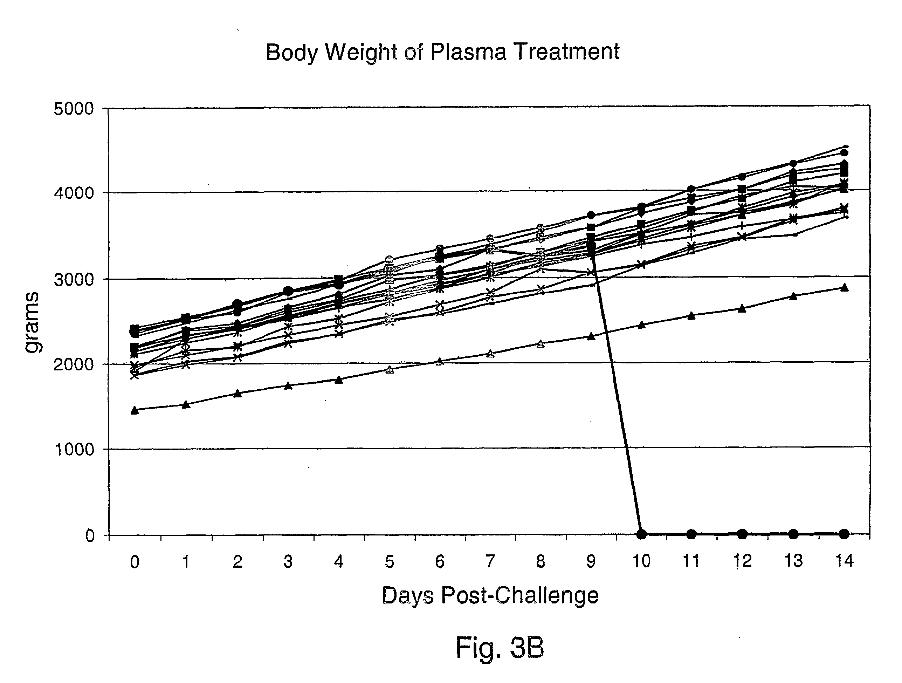

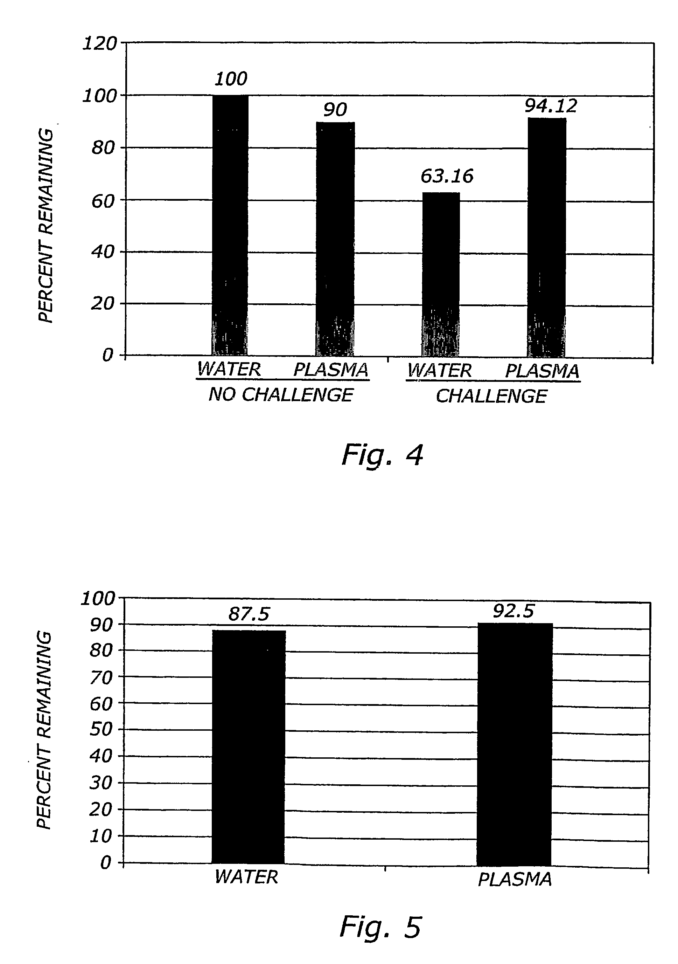

Body weight of untreated and plasma treated groups after challenge are demonstrated in Figures 3A and 3B. Seven turkeys consuming untreated water after challenge were removed or died from the challenge as depicted in Figure 3A. One turkey consuming treated water after challenge lost weight and died due to the challenge as shown in Figure 3B. Figure 4 demonstrates percent remaining after challenge, while Figure 5 demonstrates percent remaining before challenge. No differences (P > 0.05) in percent remaining were noted after the challenge period in un-challenged turkeys, while challenged turkeys consuming plasma treated water had increased (P < 0.05) percent remaining compared to challenge turkeys consuming untreated water (Figure 4). No differences (P > 0.05) were noted in percent remaining prior to challenge (d 0-35) due to consumption of treated water (Figure 5).

### Discussion

The current study demonstrates improvement in performance of turkeys during the starting period due to consumption of plasma proteins in the water. Furthermore, after a respiratory challenge, consumption of plasma proteins via the water improved survival and decreased removals. Overall, delivery of plasma proteins via the water increases feed efficiency and percent remaining (survival) after respiratory challenge and aids in starting turkeys.

### Tables

**Table 11. Main Effect of water treatment on performance in turkeys.**

| ADG | | | | |
|---|---|---|---|---|
| | Water | Plasma | SEM | P |
| D 0-7 | 14.38 | 16.62 | 0.42 | 0.0003 |
| D 7-14 | 31.64 | 32.06 | 0.69 | 0.6587 |
| D 14-21 | 50.01 | 51.18 | 1.3 | 0.5152 |
| D 21-28 | 77.53 | 78.56 | 2.18 | 0.7372 |
| D 28-35 | 98.85 | 101.85 | 3.39 | 0.5281 |
| | | | | |
| D 0-14 | 23.13 | 24.34 | 0.48 | 0.0728 |
| D 0-21 | 32.09 | 33.29 | 0.7 | 0.2212 |
| D 0-28 | 43.51 | 44.52 | 1.04 | 0.4854 |
| D 0-35 | 54.57 | 55.99 | 1.42 | 0.4772 |

| ADFI | | | | |
|---|---|---|---|---|
| | Water | Plasma | SEM | P |
| D 0-7 | 19.13 | 18.93 | 0.47 | 0.7757 |
| D 7-14 | 39.32 | 37.62 | 1.18 | 0.3361 |
| D 14-21 | 59.69 | 61.54 | 1.38 | 0.3736 |
| D 21-28 | 99.82 | 97.44 | 2.14 | 0.455 |
| D 28-35 | 162.65 | 161.66 | 4.77 | 0.8871 |
| | | | | |
| D 0-14 | 29.22 | 28.27 | 0.75 | 0.4002 |
| D 0-21 | 39.38 | 39.36 | 0.9 | 0.9889 |
| D 0-28 | 54.49 | 53.88 | 1.1 | 0.7081 |
| D 0-35 | 76.12 | 75.44 | 1.78 | 0.7922 |

| Water Disappearance | | | | |
|---|---|---|---|---|
| | Water | Plasma | SEM | P |
| D 0-7 | 68.58 | 79.8 | 3.21 | 0.0387 |
| D 7-14 | 122.25 | 131.68 | 3.29 | 0.077 |
| D 14-21 | 171.3 | 186.18 | 4.94 | 0.066 |
| D 21-28 | 236.65 | 251.42 | 8.97 | 0.2779 |
| D 28-35 | 313.1 | 339.22 | 11.59 | 0.1497 |
| | | | | |
| D 0-14 | 95.41 | 105.74 | 3 | 0.0407 |
| D 0-21 | 120.71 | 132.56 | 3.26 | 0.0332 |
| D 0-28 | 149.7 | 162.27 | 4.42 | 0.0791 |
| D 0-35 | 182.38 | 197.66 | 5.43 | 0.0819 |

| Gain/Feed | | | | |
|---|---|---|---|---|
| | Water | Plasma | SEM | P |
| D 0-7 | 0.74 | 0.88 | 0.03 | 0.0111 |
| D 7-14 | 0.79 | 0.85 | 0.01 | 0.0019 |
| D 14-21 | 0.84 | 0.83 | 0.02 | 0.9194 |
| D 21-28 | 0.76 | 0.8 | 0.01 | 0.0897 |
| D 28-35 | 0.6 | 0.63 | 0.02 | 0.2613 |
| | | | | |
| D 0-14 | 0.77 | 0.86 | 0.02 | 0.0032 |
| D 0-21 | 0.8 | 0.85 | 0.02 | 0.0544 |
| D 0-28 | 0.78 | 0.82 | 0.01 | 0.0272 |
| D 0-35 | 0.71 | 0.74 | 0.01 | 0.0618 |

**Table 12. Effect of water treatment and challenge on Performance of turkeys.**

| ADG | | | | | | | | |
|---|---|---|---|---|---|---|---|---|
| | Unchallenge | | | | Challenge | | | |
| | Water | Plasma | SEM | P | Water | Plasma | SEM | P |
| D 35-42 | 117.92 | 114.77 | 5.89 | 0.6991 | 124.06 | 135.11 | 6.09 | 0.1913 |
| D 42-49 | 123.04 | 124.58 | 5.36 | 0.8342 | 131.46 | 138.14 | 6.19 | 0.4177 |
| D 35-49 | 120.45 | 119.69 | 5.28 | 0.9167 | 129.16 | 136.65 | 6.1 | 0.3574 |

| ADFI | | | | | | | | |
|---|---|---|---|---|---|---|---|---|
| | Unchallenge | | | | Challenge | | | |
| | Water | Plasma | SEM | P | Water | Plasma | SEM | P |
| D 35-42 | 194.51 | 181.67 | 7.54 | 0.2628 | 199.56 | 208.75 | 7.54 | 0.4134 |
| D 42-49 | 242.85 | 225.3 | 14.99 | 0.4318 | 239.62 | 249.28 | 14.99 | 0.661 |
| D 35-49 | 218.69 | 203.48 | 9.72 | 0.3011 | 219.59 | 229.02 | 9.72 | 0.5124 |

| Water Disappearance | | | | | | | | |
|---|---|---|---|---|---|---|---|---|
| | Unchallenge | | | | Challenge | | | |
| | Water | Plasma | SEM | P | Water | Plasma | SEM | P |
| D 35-42 | 472.24 | 400.46 | 29.62 | 0.1096 | 459.28 | 500.85 | 29.62 | 0.3187 |
| D 42-49 | 507.57 | 516.09 | 29.22 | 0.8418 | 475.92 | 524.5 | 29.21 | 0.2735 |
| D 35-49 | 489.91 | 450.74 | 31.48 | 0.3724 | 469.52 | 512.68 | 31.48 | 0.3291 |

| Gain/Feed | | | | | | | | |
|---|---|---|---|---|---|---|---|---|
| | Unchallenge | | | | Challenge | | | |
| | Water | Plasma | SEM | P | Water | Plasma | SEM | P |
| D 35-42 | 0.6 | 0.58 | 0.02 | 0.5063 | 0.54 | 0.65 | 0.02 | 0.0149 |
| D 42-49 | 0.5 | 0.56 | 0.05 | 0.3527 | 0.48 | 0.54 | 0.05 | 0.3255 |
| D 35-49 | 0.54 | 0.57 | 0.02 | 0.4125 | 0.51 | 0.59 | 0.02 | 0.0319 |

### EXAMPLE 6

### Immunological and Gut Microbial Responses of Senior Dogs to Supplemental Spray Dried Plasma

### Materials and Methods

*Animals and diets..* Forty senior dogs with beagle (11-12 yr) or pointer (7-12 yr) bloodlines were used in this experiment. Dogs were individually housed in indoor pens with access to individual outdoor runs (approximately 1.2 x 1.5 m indoors and 1.2 x 3.0 m outdoors) in an environmentally controlled facility. All dogs were allowed free access to water while indoors.

Dogs were fed an extruded kibbled diet with poultry meal as the primary protein source and brewers rice as the primary carbohydrate source. See Table 13.

**Table 13. Ingredient composition of the basal mix and chemical composition of experimental diets fed to senior dogs¹.**

| Ingredient | | | | % in basal mix |
|---|---|---|---|---|
| Brewers rice | | | | 44.5 |
| Poultry by-product meal | | | | 32.1 |
| Poultry fat² | | | | 15.7 |
| Beet pulp | | | | 4.0 |
| Egg, dehydrated | | | | 2.24 |
| Salt | | | | 0.65 |
| KCl | | | | 0.43 |
| Choline chloride | | | | 0.13 |
| Mineral premix³ | | | | 0.12 |
| Vitamin premix⁴ | | | | 0.12 |
| Analyzed composition | 0% SDP | 0.5% SDP | 1.0% SDP | 2%SDP |
| Dry matter, %⁵ | 93.6 | 93.0 | 93.0 | 91.9 |
| | | % of dry matter | | |
| Organic matter⁵ | 90.7 | 90.5 | 90.8 | 90.8 |
| Crude protein⁶ | 33.0 | 34.2 | 33.9 | 34.6 |
| Fat⁷ | 25.4 | 25.2 | 25.4 | 25.3 |

| | | | | |
|---|---|---|---|---|
| ¹After extrusion of the base mix, the appropriate amount of spray dried plasma was solubilized in poultry fat and mixed with the basal mix in a ribbon mixer. ²10% poultry fat added internally to the basal mix and 5.7% added externally. ³Provided the following per kg of premix: 2 g Co, 10 g Cu, 1.25 g I, 75 g Fe, 10 g Mn, 0.2 g Se, and 100 g Zn. ⁴Provided the following per kg of premix: 12,474,000 IU vitamin A, 748,440 IU vitamin D, 49,896 IU vitamin E, 15 mg vitamin B₁₂, 499 mg menadione, 90.2 mg biotin, 199,584 mg choline, 598.8 mg folic acid, 37,422 mg niacin, 14,969 mg d-pantothenic acid, 9,979 mg vitamin B₆, 7,984 mg riboflavin, and 9,979 mg thiamin. ⁵Analyzed according to AOAC (1984). ⁶Analyzed via Leco analysis (AOAC, 1995). ⁷Total lipid analyzed via acid hydrolysis followed by ether extraction (AACC,1983; Budde, 1952). | | | | |

Diets contained approximately 34% crude protein and 25% fat. Spray dried plasma was solubilized in poultry fat and added to the exterior of the extruded kibble at the following concentrations: 0, 0.5, 1, and 2 % SDP. Beagles were offered 500 g and pointers 600 g of their respective diet daily.

*Experimental design and sampling procedures.* Dogs were randomly assigned to dietary treatment in a parallel design. Prior to treatment administration, baseline blood samples were collected via jugular or radial puncture into evacuated tubes containing EDTA for complete blood count (CBC) and white blood cell differentiation (neutrophil, eosinophil, basophil, lymphocyte, and monocyte). In addition, a freshly voided fecal sample was collected within 15 min of defecation and a subsample was immediately placed in a pre-weighed Carey-Blair transport media container (Meridian Diagnostics, Inc., Cincinnati, OH) for subsequent enumeration of the following bacteria: bifidobacteria, lactobacilli, *Escherichia coli,* and *Clostridium perfringens.*

After a 7 day dietary adaptation period, dogs were administered a vaccine challenge in an attempt to determine the effects of SDP on the immune capacity of the senior dog. Challenge period day 0 blood samples were collected from all dogs prior to vaccination. All dogs were then administered one, 1 ml dose, subcutaneously, of a vaccine containing canine distemper, adenovirus, type 2-coronavirus, parainfluenza, and parvovirus vaccines and leptospira bacterin (Duramune. Max 5-CvK/4L, Fort Dodge Laboratories, Inc., Fort Dodge, Iowa). Dogs had not received this vaccination previously for a minimum of 12 months. Subsequent blood samples were collected at 2, 4, 6, 8, 10, and 21 day post-vaccination via jugular or radial puncture into evacuated tubes containing either 1) EDTA for a complete blood count (CBC) and white blood cell differentiation, or 2) no anticoagulant for serum anti-canine parvovirus antibody quantification.

*Chemical analyses.* Microbial concentrations were determined in fresh fecal samples by serial dilution in anaerobic diluent before inoculation onto respective Petri dishes of sterile agar. The selective medium for bifidobacteria spp. was anaerobically prepared using BIM-25 agar (BBL Microbiology Systems, Cockeyville, MD) according to the method described by Muñoa and Pares (1988). Lactobacilli were cultured on Rogosa SL agar (Difco Laboratories, Detroit, MI). *Escherichia coli* was enumerated using EMB agar (Difco Laboratories, Detroit, MI), while *C*. *perfringens* was enumerated on a tryptose-sulfite-cycloserine (TSC) agar with egg yolk (FDA, 1992).

All agars were inoculated using a repeating pipette to dispense 7 drops of 10 µl each of the appropriate dilutions. After the drops adsorbed to the agar, plates were inverted and incubated at 38°C either anaerobically (bifidobacteria, lactobacilli, and *C*. *perfringens;* 75% N2, 20% CO2, 5% H2) or aerobically (*E. coli*). Colony counts were made after 24 to 48 h of incubation. A colony-forming unit (CFU) was defined as a distinct colony measuring at least 1 mm in diameter.

Complete blood counts and white blood cell differential distributions (neutrophils, lymphocytes, monocytes, eosinophils, and basophils), were determined on whole blood samples. In addition, blood collected in anticoagulant-free tubes was centrifuged at 2060 x g for 20 min at 4° C. Serum samples were stored at -80° C until quantification of anti-canine parvovirus antibodies via a hemagglutination inhibition tests (Carmichael et al., 1980).

*Statistical analyses.* Data were analyzed as a completely randomized design using the General Linear Models procedure of SAS (SAS Institute, Cary, NC). Since the quantity of feed offered to the beagles differed from the pointers, statistical analysis was done within breed. Individual animal weight was used as a covariate in feed intake analysis. Pretreatment bacterial and challenge day 0 blood analysis data also were used as a covariate in appropriate post-treatment data analyses. Comparison between the control and individual SDP treatments was made using non-orthogonal contrasts. Comparisons were considered statistically different at P < 0.05. Comparisons with a P = 0.06 to 0.15 were considered trends due to the variability associated with many of the criteria evaluated.

### Results and Discussion

*Food intake.* The effect of SDP on food intake was evaluated during the baseline (final 3 day), immediate post-vaccination (day 0 through 6), and overall challenge (day 0 through 21) periods (Table 14).

**Table 14. As-is food intake of senior dogs fed an extruded diet supplemented with 0, 0.5, 1, or 2% spray dried plasma¹**

| | SDP, % of diet | | | | | Contrast P value² | | |
|---|---|---|---|---|---|---|---|---|
| Period | 0 | 0.5 | 1.0 | 2.0 | SEM | Control vs Plasma | Linear | Quadratic |
| Beagle food intake, g/d | | | | | | | | |
| Baseline³ | 343 | 342 | 365 | 351 | 43.8 | NS | NS | NS |
| Day 0-6⁴ | 194 | 194 | 197 | 223 | 17.7 | NS | NS | NS |
| Day 0-21⁵ | 192 | 157 | 209 | 213 | 15.7 | NS | 0.13 | NS |
| Pointer food intake, g/d | | | | | | | | |
| Baseline³ | 597 | 555 | 541 | 596 | 24.9 | NS | NS | 0.06 |
| Day 0-6⁴ | 449 | 456 | 438 | 520 | 35.0 | NS | NS | NS |
| Day 0-21⁵ | 417 | 417 | 377 | 457 | 30.8 | NS | NS | NS |

| | | | | | | | | |
|---|---|---|---|---|---|---|---|---|
| ¹Values are 1smeans. ²NS = Not significantly different (P > 0.15). ³Average food intake of last 3 d of the baseline period. ⁴Average food intake of d 0 to 6 post vaccination. ⁵Average food intake of d 0 to 21 post vaccination. | | | | | | | | |

Inclusion of SDP did not affect baseline food intake in the beagles. During the baseline period, pointers fed diets containing either 0.5 or 1.0% SDP had lower food intake than pointers fed the control or 2% SDP diets, resulting in a quadratic effect (P = 0.06) of SDP supplementation on food intake. During the immediate post-vaccination and overall challenge periods, mean food intake of dogs on all treatments decreased. This may have been due to an immunological response to the vaccination or to the increased handling of the dogs for blood collection during this period. Supplementation of SDP did not affect food intake of pointers during immediate post-vaccination or overall challenge periods and tended (P = 0.13) to linearly increase food intake of beagles during the overall challenge period.

*Microbial populations.* Results of the fecal microbial analyses are presented in Table 15 below.

**Table 15. Select fecal microbial concentrations for senior dogs fed an extruded diet supplemented with 0, 0.5, 1, or 2% spray dried plasma¹.**

| | SDP, % of diet | | | | | Contrast P value² | | |
|---|---|---|---|---|---|---|---|---|
| Item | 0 | 0.5 | 1.0 | 2.0 | SEM | Control vs Plasma | Linear | Quadratic |
| Bifidobacteria | 10.5 | 10.4 | 10.2 | 10.3 | 0.13 | 0.13 | NS | NS |
| Lactobacilli | 9.4 | 10.0 | 9.2 | 9.2 | 0.22 | NS | NS | NS |
| *Escherichia coli* | 7.9 | 8.1 | 7.2 | 7.4 | 0.36 | NS | NS | NS |
| *Clostridium perfringens* | 10.0 | 10.1 | 10.1 | 10.2 | 0.16 | NS | NS | NS |

| | | | | | | | | |
|---|---|---|---|---|---|---|---|---|
| ¹Lsmeans are expressed as log10 colony-forming units/g dry feces. ²NS = Not significantly different (P > 0.15). | | | | | | | | |

Overall, dietary supplementation with SDP did not affect (P > 0.05) fecal concentrations of lactobacilli but tended (P=0.13) to decrease the concentration of bifidobacteria compared to control dogs. Microbial populations of the gut play a critical role in development of the immune system, resistance to pathogenic bacteria, and short chain fatty acid (SCFA) production in the gut. The presence of particular microbial populations may be either beneficial or harmful to the host. Both bifidobacteria and lactobacilli are beneficial bacterial populations in the gastrointestinal tract. Increase concentrations of these organisms have been associated with decreased fecal concentrations of potentially pathogenic bacteria (Araya-Kojima et al., 1995; Gibson and Wang, 1994) and decreased concentrations of carcinogenic and putrefactive compounds in digesta (Hara et al., 1994; Mitsuoka, 1982). Although SDP tended to decrease the concentration of bifidobacteria, numerically the differences were small and of questionable biological significance.

Fecal concentrations ofE. *coli* and *C. perfringens* were not different (P > 0.05) among treatment groups. *Escherichia coli* exhibits both harmful (i.e., production of carcinogens) and beneficial (i.e., stimulation of immune function) effects in the gut (Gibson and Roberfroid,1995). A reduction in the number of clostridia in the gut is beneficial to the dog since this organism is pathogenic and can exert harmful effects on the host (Gibson and Roberfroid, 1995). Overall, as assessed in this study, SDP did not significantly impact the microbial ecology of the gut.

*Immune characteristics.* In an effort to determine the effect of SDP on the immune capacity of the senior dog, white blood cell and antibody production responses to a vaccination challenge were determined. Although it had been over a year since these dogs last were vaccinated for parvovirus, 28 dogs had very high (1:80 to 1:320) pre-vaccination antibody titers. It has been determined that an antibody titer of 1:80 or greater to parvovirus is protective (Murphy et al., 1999). Since the potential for effects of SDP dietary supplementation on antibody titer response may have been reduced in these animals, and they were already in a state of protection, their data were not included in the statistical analysis of white blood cell distribution or parvovirus antibody titers.

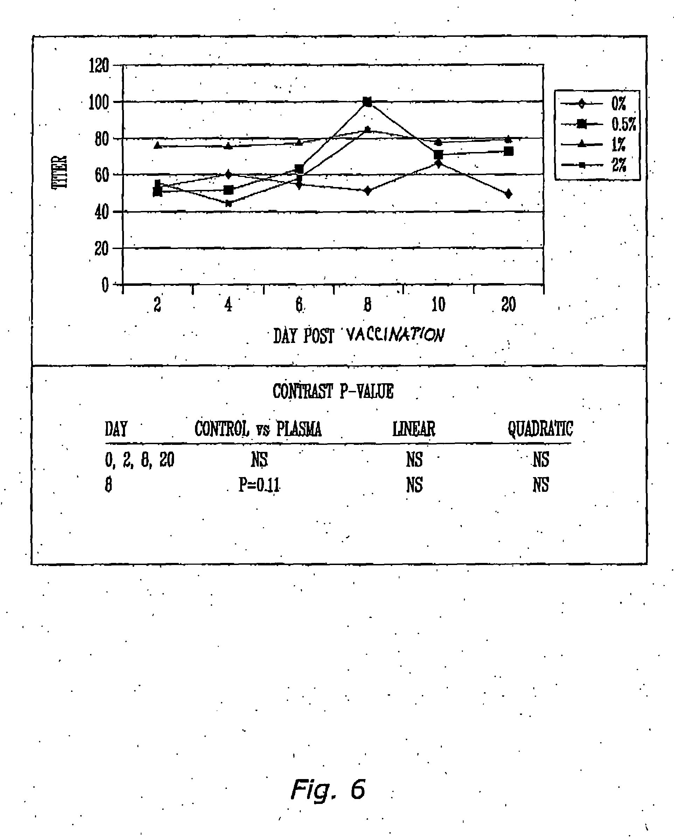

The effects of SDP supplementation on canine parvovirus hemagluttination titers are presented in Figure 6. Spray dried plasma supplementation did not affect titer level on day 2, 4, 6, 10, or 20, but on day 8 dogs supplemented with SDP tended (P = 0.11) to have a greater titer than non-supplemented dogs. These results imply that on d 8 dogs supplemented with SDP tended to have a greater capacity to mount an immune response to this vaccine, or to an actual parvovirus challenge had that occurred.

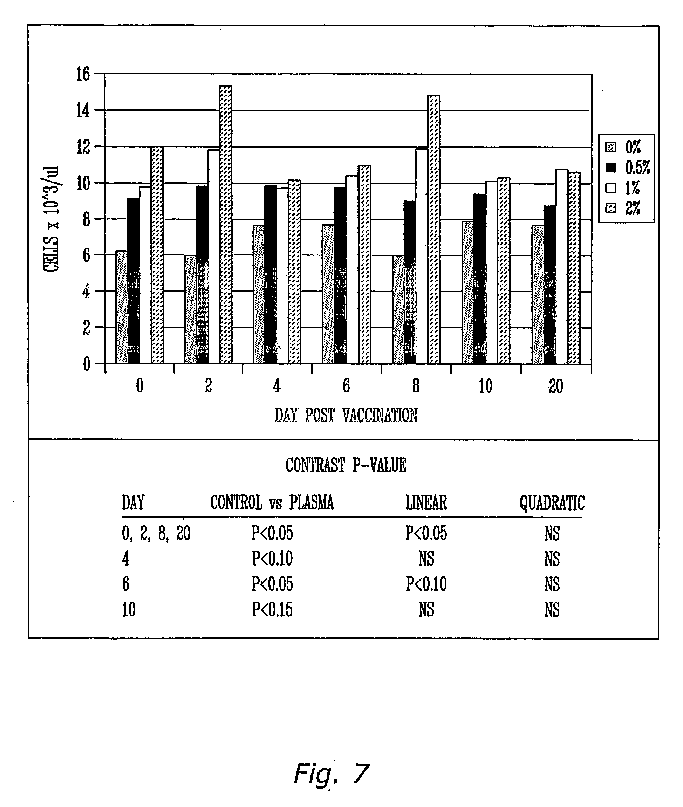

Total white blood cell, neutrophil, and lymphocyte numbers decrease with aging in the dog (Strasser et al., 1993), resulting in a potential reduction in the immunological defenses of the elderly dog. The effects of SDP supplementation on peripheral white blood cell concentrations for senior dogs are presented in Figure 7. After the 7 day dietary adaptation phase, SDP supplementation increased the challenge period day 0 WBC concentrations linearly (P < 0 .05). This differential was enhanced by vaccination, such that at 2 day post-vaccination, dogs supplemented with 2% SDP had a 61 % greater concentration of WBC compared to control dogs. Linear increases (P < 0.05) in WBC concentrations due to SDP supplementation were observed on days 0, 2, 8, and 20 post-vaccination. In addition, overall WBC concentrations for the control dogs were increased (P < 0.05) or tended to increase (P < 0.15) on all days compared to dogs receiving SDP supplementation.

In an effort to further characterize the effects of SDP supplementation on the peripheral WBC, differential analysis was conducted (Table 16).

**Table 16. White blood cell distribution for senior dogs fed an extruded diet supplemented with 0, 0.5, 1, or 2% spray dried plasma¹.**

| | | SDP, % of diet | | | | Contrast P value² | | |
|---|---|---|---|---|---|---|---|---|
| | | | | | | Control | Linear | Quadratic |
| Item | 0 | 0.5 | 1.0 | 2.0 | SEM | vs. Plasma | | |
| Neutrophils, cells x 10³/ul | | | | | | | | |
| Day 0 | 4.6 | 6.5 | 7.3 | 8.9 | 0.62 | 0.02 | 0.02 | NS |
| Day 2 | 4.0 | 6.7 | 8.9 | 10.9 | 1.03 | 0.02 | 0.02 | NS |
| Day 4 | 5.2 | 6.7 | 6.4 | 7.0 | 0.57 | 0.13 | NS | NS |
| Day 6 | 4.3 | 6.9 | 8.4 | 7.9 | 0.70 | 0.02 | 0.06 | 0.04 |
| Day 8 | 4.0 | 5.9 | 9.5 | 10.3 | 1.11 | 0.03 | 0.02 | NS |
| Day 10 | 5.1 | 6.4 | 7.1 | 7.5 | 0.64 | 0.09 | 0.13 | NS |
| Day 20 | 4.7 | 5.7 | 7.5 | 6.9 | 0.73 | 0.12 | NS | NS |
| Lymphocytes, cells x 10³/ul | | | | | | | | |
| Day 0 | 1.2 | 1.6 | 1.9 | 1.4 | 0.17 | 0.12 | NS | 0.07 |
| Day 2 | 1.7 | 1.7 | 2.1 | 1.4 | 0.17 | NS | NS | 0.13 |
| Day 4 | 1.2 | 1.8 | 2.3 | 1.6 | 0.18 | 0.03 | NS | 0.02 |
| Day 6 | 1.9 | 1.8 | 2.3 | 1.6 | 0.29 | NS | NS | NS |
| Day 8 | 1.7 | 1.8 | 1.8 | 1.6 | 0.21 | NS | NS | NS |
| Day 10 | 2.0 | 1.8 | 2.0 | 1.2 | 0.18 | NS | 0.05 | NS |
| Day 20 | 2.1 | 1.9 | 2.6 | 1.8 | 0.30 | NS | NS | NS |
| Monocytes, cells x 10³/ul | | | | | | | | |
| Day 0 | 0.50 | 0.52 | 0.72 | 0.65 | 0.19 | NS | NS | NS |
| Day 2 | 0.75 | 0.93 | 1.41 | 1.18 | 0.14 | 0.06 | 0.09 | 0.10 |
| Day 4 | 0.80 | 0.91 | 1.00 | 0.93 | 0.08 | NS | NS | NS |
| Day 6 | 0.67 | 0.73 | 0.80 | 0.74 | 0.17 | NS | NS | NS |
| Day 8 | 0.69 | 0.82 | 1.26 | 0.94 | 0.17 | NS | NS | NS |
| Day 10 | 0.94 | 0.81 | 1.20 | 0.86 | 0.11 | NS | NS | NS |
| Day 20 | 0.56 | 0.64 | 0.77 | 0.99 | 0.18 | NS | NS | NS |

| | | | | | | | | |
|---|---|---|---|---|---|---|---|---|
| ¹Values are lsmeans of dogs responding to vaccine challenge. On d 0, 2, 4, 6, 8, and 10, n = 3, 5, 2, and 2 for supplement levels 0, 0.5, 1, and 2%, respectively. On d 20, n = 3, 5, 2, and 1 for supplement levels 0, 0.5, 1, and 2 %, respectively. ²NS = not significantly different (P > 0.15). | | | | | | | | |

Spray dried plasma increased (P < 0.05) neutrophil concentrations on days 0, 2, 6, and 8 post-vaccination, and tended (P < 0.15) to increase neutrophil concentration on days 4, 10, and 20. In general, the increases observed were linear. Spray dried plasma tended (P = 0.12) to affect the peripheral lymphocyte concentrations on d 0 prior to vaccination and day 4 post-vaccination (P = 0.02). A linear decline (P = 0.05) in lymphocyte concentration was observed on day 10 and quadratic changes were detected on d 0 and 4 post-vaccination in dogs supplemented with SDP. Monocyte concentrations were quadratically affected by SDP supplementation on day 2 post-vaccination. At this time, dogs supplemented with 0.5 and 1.0 % SDP had increased monocyte concentrations (0.93 and 1.41 x 10³ cells/µl, respectively compared to control dogs (0.75 x 10³ cells/µl), while dogs supplemented with 2.0% SDP had a slightly lower concentration (1.18 x 10³ cells/µl). No differences (P > 0.05) were observed in peripheral monocyte concentration on any other day during the challenge period. These data demonstrate that SDP clearly affected the concentration of WBC, particularly after an immune challenge.

## Claims

1. Use of animal plasma from an animal source in the manufacture of a medicament for oral administration for enhancing the immune function in senior canines.

2. The use of claim 1 wherein said animal plasma administered is spray-dried plasma.

3. The use of claim 2 wherein said animal plasma is in a solubilized form.

4. The use of claim 3 wherein said animal plasma is added to a food source.

5. The use of claim 1 wherein the source of the animal plasma is selected from the group consisting of pig, bovine, ovine, poultry, equine and goat plasma.

6. The use of claim 1 wherein said canine is from about 7 to about 12 years of age.

7. Animal plasma from an animal source for use in enhancing the immune function in senior canines wherein said animal plasma is for oral administration.

## Patentansprüche

1. Verwendung von Tierplasma tierischen Ursprungs bei der Herstellung eines Medikaments für die orale Verabreichung zur Stärkung der Immunfunktion bei älteren Hunden.

2. Verwendung nach Anspruch 1, wobei das verabreichte Tierplasma sprühgetrocknetes Plasma ist.

3. Verwendung nach Anspruch 2, wobei das Tierplasma in einer solubilisierten Form vorliegt.

4. Verwendung nach Anspruch 3, wobei das Tierplasma einem Futter zugegeben wird.

5. Verwendung nach Anpruch 1, wobei die Herkunft des Tierplasmas ausgewählt ist unter Schweine-, Rinder-, Schaf-, Geflügel-, Pferde- und Ziegenplasma.

6. Verwendung nach Anspruch 1, wobei der Hund etwa 7 bis etwa 12 Jahre alt ist.

7. Tierplasma tierischen Ursprungs zur Verwendung bei der Stärkung der Immunfunktion bei älteren Hunden, wobei das Tierplasma für die orale Verabreichung bestimmt ist.

## Revendications

1. Utilisation de plasma animal d'une source animale pour la fabrication d'un médicament destiné à l'administration orale pour renforcer la fonction immunitaire chez les chiens âgés.

2. Utilisation selon la revendication 1, dans laquelle ledit plasma animal administré est du plasma séché par atomisation.

3. Utilisation selon la revendication 2, dans laquelle ledit plasma animal est sous forme solubilisée.

4. Utilisation selon la revendication 3, dans laquelle ledit plasma animal est ajouté à une source alimentaire.

5. Utilisation selon la revendication 1, dans laquelle la source du plasma animal est choisie dans le groupe consistant en plasma de porc, de boeuf, de mouton, de volaille, de cheval et de chèvre.

6. Utilisation selon la revendication 1, dans laquelle ledit chien est âgé d'environ 7 à environ 12 ans.

7. Plasma animal d'une source animale pour utilisation en vue de renforcer la fonction immunitaire chez des chiens âgés, ledit plasma animal étant destiné à une administration orale.
